# EUROPEAN PATENT SPECIFICATION

(11) **EP 0 802 894 B1**
(45) Date of publication and mention of the grant of the patent: **28.04.1999**
(21) Application number: 96900607.1
(22) Date of filing: 11.01.1996
(51) Int. Cl.: C07C 43/04, C07C 41/06, C07C 41/42

(54) **PROCESS FOR PREPARING ALKYL TERTIARY-ALKYL ETHER**
VERFAHREN ZUR HERSTELLUNG VON ALKYL-TERTIÄR-ALKYLETHERN
PROCEDE DE PREPARATION D'ETHER D'ALKYLE-ALKYLE TERTIAIRE

(30) Priority: 13.01.1995 EP 95200086
(43) Date of publication of application: 29.10.1997
(73) Proprietor: SHELL INTERNATIONALE RESEARCH MAATSCHAPPIJ B.V., 2596 HR Den Haag (NL)
(72) Inventor: AMESZ, Dirk, Jacob, NL-2596 HR The Hague (NL); MOOLDIJK, Robertus, Jan, NL-2596 HR The Hague (NL); SIMONS, Theodoor, Johan, Leonard, Wenceslaus, NL-2596 HR The Hague (NL)
(86) International application number: EP9600264
(87) International publication number: WO9621635

(56) References cited:
- EP-A- 0 068 514
- EP-A- 0 078 422
- EP-A- 0 323 134
- DE-A- 2 705 538
- GB-A- 2 010 323
- US-A- 4 198 530

## Description

The invention relates to a process for preparing alkyl tertiary-alkyl ethers such as methyl tert-butyl ether ("MTBE"), ethyl tert-butyl ether ("ETBE"), tert-amyl methyl ether ("TAME"), etc. Such ethers are popular high-octane additives for liquid fuels, especially gasoline, replacing the environmentally controversial organolead antiknock compounds.

Processes for preparing MTBE and other ethers are known in the art. Thus, they may be made by reacting an alcohol (typically a primary alcohol such as methanol, hereinafter "MeOH") with an iso-olefin (e.g., isobutene) or the corresponding tertiary alcohol (e.g., tert-butanol) in the presence of an acid catalyst. As 100% conversion is not achieved, the reaction mixture comprises the product ether but also starting reagents. Unfortunately, MTBE and MeOH are hard to separate by distillation as an MeOH/MTBE azeotrope is formed.

Initially, the product ethers were separated from the reaction mixture by washing and/or distilling (repeatedly, under pressure or combinations thereof). Subsequently, in German patent application No. 2,705,538 and US patent No. 4,198,530 it is taught to distil in the presence of a hydrocarbon, whereby a new azeotrope of hydrocarbons and MeOH is formed and separated. A next improvement is taught in European patent application No. 78,422, wherein an amount of hydrocarbons is used that is higher than the content in the hydrocarbons/MeOH azeotrope, thereby resulting in the separation of 99% pure or more MTBE. In the processes of each of these specifications, the hydrocarbons/MeOH azeotrope is separated into its components MeOH, respectively hydrocarbons. Thus, an additional separation unit (washing unit, fractionator, etc.) is required. This makes these processes complex and less attractive. In case the elevated temperature of the azeotrope is not used, these processes are even less attractive for economical reasons. Therefore, it remains desirable to find a process for preparing alkyl tertiary-alkyl ethers in an economically attractive manner, which process allows simple recovery of high purity product streams produced at low energy consumption.

From U.K. patent application No. 2,010,323 a process for preparing gasoline containing tertiary amyl methyl ether is known, which process comprises the steps of
(1) separating from a mixture of hydrocarbons a portion of olefinic hydrocarbons having predominantly 5 carbon atoms,
(2) catalytically etherifying the olefinic hydrocarbons with methanol,
(3) fractionally distilling under reflux at least part of the effluent stream from step (2), thereby withdrawing a bottom fraction containing a major proportion of the ethers and a distillate fraction,
(4) blending the bottom fraction and any remainder of the effluent stream from step (2) with gasoline, and
(5) recycling part of the distillate fraction to the catalytic reaction of step (2) and blending a part of the distillate fraction with the gasoline when the entire effluent from step (2) is passed to step (3).

It has now been discovered that this objective may be achieved by heating at least part of the iso-olefin reagent in the distillation column, where it assists in fractionating the reaction mixture, and using at least part of the overhead mixture (product obtained at the top of the distillation column, containing the iso-olefin reagent and alcohol) without separation into its components, to feed the reaction. Accordingly, the invention provides a process for preparing an alkyl tertiary-alkyl ether by
(a) feeding an iso-olefin reagent-containing stream and an alcohol reagent to a reactor and allowing these reagents to react under etherification conditions to form an alkyl tertiary-alkyl ether-containing reaction mixture,
(b) passing the reaction mixture produced in (a) and a feed stream comprising C4 to C9 hydrocarbons (hereinafter, the "hydrocarbon feed stream") to a distillation column,
(c) fractionating the reaction mixture and hydrocarbons to obtain a bottom product comprising substantially pure alkyl tertiary-alkyl ether and an overhead mixture comprising alcohol and hydrocarbons, and
(d) recycling at least part of the overhead mixture to the reactor,
wherein the hydrocarbon feed stream comprises iso-olefin reagent, wherein at least part of the iso-olefin reagent-containing stream supplied into the reactor is formed by the overhead mixture (hereinafter, the "distillation recycle"), and wherein the hydrocarbon feed stream is passed into the distillation column through a separate inlet located below that of the reaction mixture.

The iso-olefin reagent-containing stream is formed by the distillation recycle and, optionally, a stream of fresh reagent. Preferably (since economically more attractive), at least 50 %wt, more preferably at least 80 %wt, of the iso-olefin reagent-containing stream is formed by the distillation recycle. Most preferably, all of the iso-olefin reagent that is fed into the reactor stems from the distillation recycle.

Besides the iso-olefin, the hydrocarbon feed stream may further comprise inert hydrocarbons such as butane, pentane, etc., and essentially inert hydrocarbons such as 1-butene, etc. Preferably, the hydrocarbon feed stream comprises at least 50 %wt of the iso-olefin reagent. To avoid build-up of inert hydrocarbons, a bleed stream may be introduced downstream of the reactor and/or the distillation column. Optionally, a conventional isomerization unit (as disclosed in for instance US patents Nos. 5,243,090, 5,254,748 and 5,254,764) may be used to isomerize (part of) the inert hydrocarbons into the iso-olefin reagent. There is no advantage in using hydrocarbon feed streams comprising C3- or C9+ hydrocarbons as these components do not facilitate the fractionation of the reaction mixture in the distillation column.

More preferably the hydrocarbon feed stream comprises at least 80 %wt, most preferably at least 99 %wt of the iso-olefin reagent. Build-up of inert hydrocarbons in this most preferred embodiment is very small, if any. Accordingly, the overhead mixture may be recycled in total to the reactor without need of a bleed stream. Loss of reagents/products is then negligible, whereas the elevated temperature of this overhead mixture is optimally used.

The hydrocarbon feed stream is passed into the distillation column through a separate inlet that is located below the inlet of the reaction mixture, whereby nearly all of the alcohol reagent may be removed from the bottom product comprising the alkyl tertiary-alkyl ether.

Preferably, the amount of hydrocarbon feed stream is such as to introduce at least sufficient hydrocarbons into the distillation column to separate the alcohol in the form of an azeotrope of the alcohol and the hydrocarbons from the produced alkyl tertiary-alkyl ether. This amount is related to the internal pressure in the distillation column. For instance, from US patent No. 4,198,530 it is known that at a pressure of 5 atm (5.07 bar a), the MeOH contents in the azeotrope is about 4 mol%, but at a pressure of 10 atm (10.1 bar a), it is about 9 mol%. The amount is also related to the content of hydrocarbons in the reaction mixture, for instance, of excess iso-olefin reagent and inert hydrocarbons due to build-up, if any.

Suitable iso-olefin reagents used in the present process have up to 8 carbon atoms and are more suitably selected from isobutene and isoamylenes (2-methyl-1-butene, 2-methyl-2-butene and dimethylpropene).

The alcohol reagent suitably has up to 8 carbon atoms and is more suitable a primary alcohol, such as methanol and ethanol, but may also be a secondary, tertiary or aromatic alcohol. Tertiary-alkyl ethers of primary alcohols generally have the better combustion properties and are therefore preferred.

Preferably, the molar ratio wherein the iso-olefin reagent and the alcohol reagent are allowed to react ranges from 1:0.9 to 1:1.1, and is more preferably about 1:1.02. Using more than the stoichiometric amount of one of the reagents allows improved conversion in respect of the other reagent. However, in case excess alcohol reagent is used, the difficulty in obtaining sufficiently pure alkyl tertiary-alkyl ether is aggrevated, whereas in case excess iso-olefin reagent is used, large volumes thereof will be recirculated, increasing the costs of equipment.

The catalyst typically is an acidic solid catalyst such as a sulfonic acid substituted ion exchange resin or an acidic natural or synthetic silicate (e.g., amorphous silica-alumina or acid zeolites). Preferably, the catalyst is an ion exchange resin, e.g., produced by polymerization of aromatic vinyl compounds to which catalytically active functional groups are covalently bonded. Suitable sulfonic acid substituted ion exchange resins are disclosed in European patent application No. 102,840 and in International application No. 90/08758. A very suitable catalyst is a sulfonic acid substituted ion exchange resin having at least 1.2, more preferably about 1.2 to 1.8 sulfonic acid groups for each aromatic ring system. Typical examples of very suitable catalysts are sulfonic acid substituted divinylbenzene-styrene resins sold under the trademarks DUOLITE C20, DUOLITE C26, AMBERLYST 15, AMBERLITE IR-120, AMBERLITE 200 and DOWEX 50 (DUOLITE, AMBERLITE and DOWEX are trade marks).

The reaction usually is conducted at a temperature between 30 and 120 °C, preferably between 45 and 100 °C, and a pressure between 5 and 40 bar g, preferably between 10 and 20 bar g. Typically, the reagents are supplied and made to flow continuously through the sulfonic acid substituted ion exchange resin which is disposed in the form of a fixed bed, at a liquid hourly space velocity (LHSV) that is generally less than 80 hr⁻¹. The conditions are all known to those skilled in the art. Indeed, similar conditions and catalysts may be used without departing from the gist of the invention.

The distillation is suitably carried out in a distillation tower having 30 to 50 theoretical plates ("TPs") that is, for instance, operated at a pressure between 6 and 12 bar g, a bottom temperature (T_{B}) between 130 and 150 °C, and a top temperature (T_{T}) between 50 and 70 °C. The invention may quite suitably be carried out with a distillation tower of 40 TPs, operating at a pressure of 9 bar g, a T_{B} of 140 °C, and a T_{T} of 65 °C (all approximations).

In a preferred embodiment of the invention, the reaction mixture is employed in indirect heat transfer with the (hot) bottom product comprising the alkyl tertiary-alkyl ether. In a more preferred embodiment the bottom product is also employed in indirect heat transfer with the hydrocarbon feed stream further downstream of the distillation column.

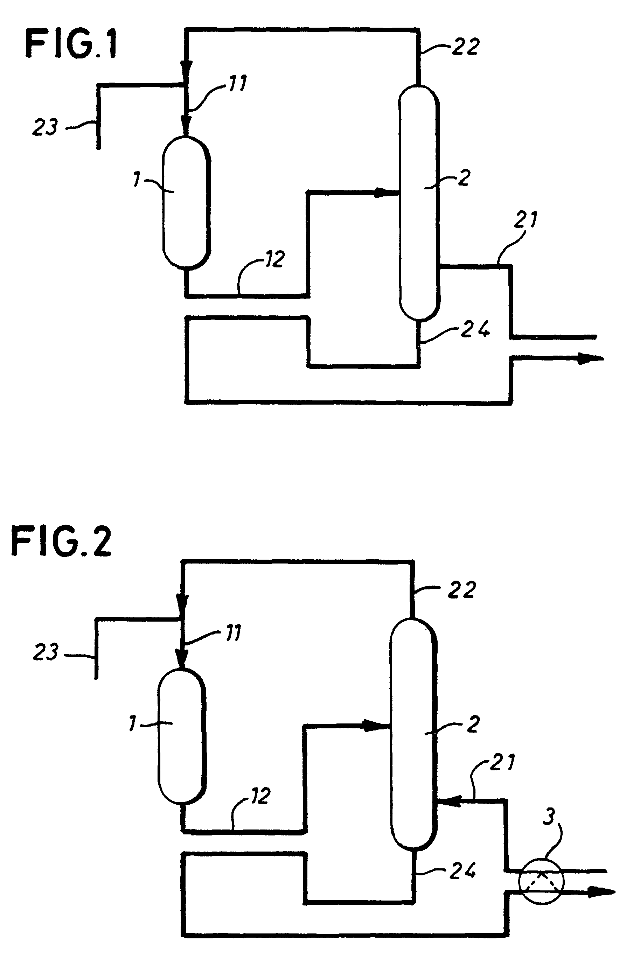

Referring now to Figure 1, a schematic process flow diagram is presented for a specific embodiment (A) of the instant invention. In this embodiment and subsequent embodiments of the present invention, the hydrocarbon feed stream, introduced via inlet 21, is composed of isobutene.

In Figure 1, the reagents introduced via inlet 11 essentially comprise isobutene as iso-olefin and MeOH as primary alcohol in a molar ratio of 1:1.02. It is passed to reactor 1 containing an acid catalyst (e.g., "AMBERLYST 15") for etherification at an LHSV of about 20 hr⁻¹, a pressure of 20 bar g and a temperature of 50 °C. Via pipe 12, the reaction mixture, essentially comprising isobutene, MeOH and MTBE (conversion of about 99%), is passed to the mid-portion of distillation column 2 (a column counting 37 TPs). The distillation is carried out at an internal pressure of 9 bar g, a T_{B} of 141 °C and a T_{T} of 63 °C. The inlet 21 of the hydrocarbon feed stream is below the inlet of the reaction mixture. The amount of hydrocarbon feed stream is sufficient to compensate the amount of isobutene converted into MTBE (about 40,000 kg (40 ton) per hour). The overhead mixture obtained at the top of the distillation column 2, via outlet 22, is essentially composed of isobutene and MeOH (about 47,000 kg (47 ton) per hour, ratio isobutene to MeOH of about 97:3 m/m). An alcoholic feed stream of fresh MeOH is introduced via inlet 23 in an amount sufficient to change the molar ratio to that of the reaction feedstock (about 23,000 kg (23 ton) per hour). The bottom product, obtained at outlet 24 is essentially composed of the alkyl tertiary-alkyl ether, MTBE (about 63,000 kg (63 ton) per hour).

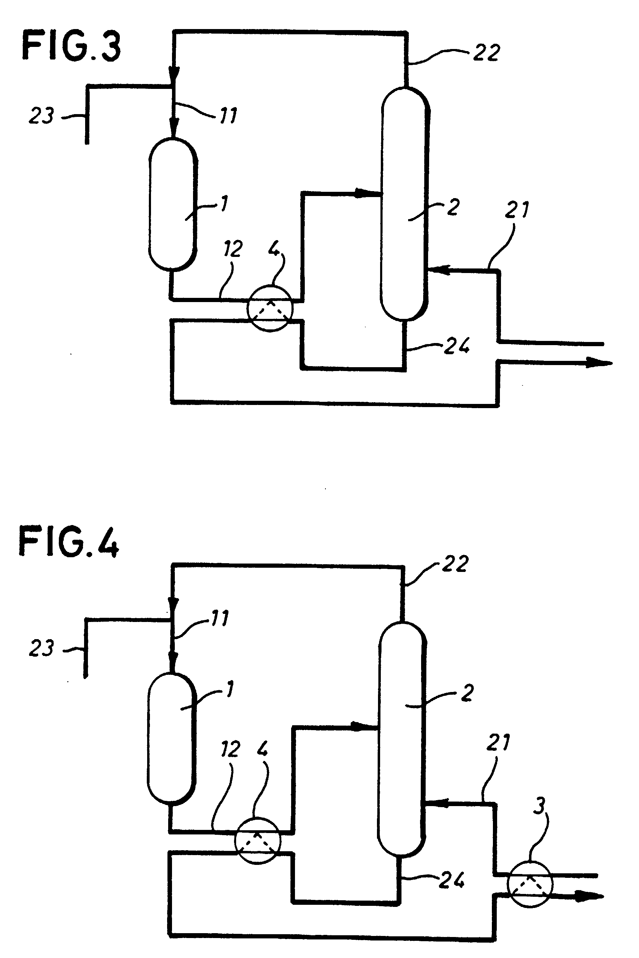

A preferred embodiment (B) of the present invention is illustrated as a schematic diagram in Figure 2. In Figure 2, the bottom product, obtained at outlet 24, is cooled in heat exchanger 3 by indirect heat exchange with the hydrocarbon feed stream, introduced via inlet 21. In an alternative embodiment (C) (Figure 3), the bottom product is cooled in heat exchanger 4 by indirect heat exchange with the reaction mixture, transported via pipe 12. In the most preferred embodiment (D) of the present invention (Figure 4), the bottom product is first cooled in heat exchanger 4 by indirect heat exchange with the reaction mixture and subsequently (downstream) in heat exchanger 3 with the hydrocarbon feed stream.

The virtue of each of the embodiments (A) to (D) is that no extraction of recycle methanol is required. In addition, the labour (i.e., energy consumption per second, expressed in Joules per second (= Watts)) required to prepare 1000 kg of MTBE per hour for embodiment (A) is small, and that of embodiments (B) to (D) smaller still. The results of a computer simulation have been summarized in the Table.

The Table is completed with results of computer simulations that are based on a process (C1) wherein the iso-olefin reagent is heated to the same temperature as in embodiments (A) to (D), however now heating the iso-olefin externally (requiring about 1 MJ/s (1 MW) to bring the 40 ton isobutene to the same temperature as that of the distillation recycle). Moreover, the distillation column is fed with a separate flow of liquid hydrocarbons. The overhead mixture is then led to a separation unit (i.e., a washing unit with a duty of about 3.5 MJ/s (3.5 MW)) where the alcohol and the liquid hydrocarbons are separated. The alcohol is recirculated to the reactor, the liquid hydrocarbons are reused in the distillation column. Other conditions are kept identical to those of embodiment (A). Also included are the results based on a process (C2) similar to that of (C1), wherein the bottom product is in indirect heat exchange with the reaction mixture (cf. European patent application No. 0,323,134, Fig. 1 or 2). Other conditions are kept identical to those of embodiment (C).

Advantageously, in the present process no separation unit for the methanol is needed. Moreover, the present process is clearly more energy-efficient (less energy per 1000 kg (ton) MTBE per hour).

**Table**

| Embodiment | A | B | C | D | C1 | C2 |
|---|---|---|---|---|---|---|
| Duty of column 2 (in MJ/s (= MW)) | 13.1 | 13.1 | 13.1 | 13.1 | 13.1 | 13.1 |
| Heat recovery in 3 (in MJ/s (=(MW)) | n.a. | 1.9 | n.a. | 1.9 | n.a. | 1.9 |
| Heat recovery in 4 (in MJ/s (=(MW)) | n.a. | n.a. | 2.0 | 1.7 | n.a. | n.a. |
| External heating of isobutene (in MJ/s (= MW)) | n.a. | n.a. | n.a. | n.a. | 1.0 | 1.0 |
| Duty of additional separation unit (in MJ/s (=MW))¹⁾ | n.a. | n.a. | n.a. | n.a. | 3.5 | 3.5 |
| Total duty (in MJ/s (=(MW))²⁾ | 13.1 | 11.2 | 11.1 | 9.5 | 17.6 | 15.7 |
| Energy efficiency (in kJ/s per 1000 kg MTBE (=kW/ton MTBE)) | 208 | 178 | 176 | 150 | 280 | 250 |

| | | | | | | |
|---|---|---|---|---|---|---|
| ¹⁾ Estimated value, based on heats of vaporisation | | | | | | |
| ²⁾ Relative value, based on identical conditions and parameters n.a. Not applicable | | | | | | |

## Claims

1. A process for preparing an alkyl tertiary-alkyl ether by
(a) feeding an iso-olefin reagent-containing stream and an alcohol reagent to a reactor and allowing these reagents to react under etherification conditions to form an alkyl tertiary-alkyl ether-containing reaction mixture,
(b) passing the reaction mixture produced in (a) and a feed stream comprising C4 to C9 hydrocarbons (hereinafter, the "hydrocarbon feed stream") to a distillation column,
(c) fractionating the reaction mixture and hydrocarbons to obtain a stream comprising substantially pure alkyl tertiary-alkyl ether at the bottom of the distillation column and a stream comprising alcohol and hydrocarbons at the top of the distillation column, and
(d) recycling at least part of the overhead mixture to the reactor,
wherein the hydrocarbon feed stream comprises iso-olefin reagent, wherein at least part of the iso-olefin reagent-containing stream supplied into the reactor is formed by the overhead mixture, and wherein the hydrocarbon feed stream is passed into the distillation column through a separate inlet located below that of the reaction mixture.

2. A process as claimed in claim 1, wherein at least 50 %wt of the iso-olefin reagent-containing stream is formed by the overhead mixture.

3. A process as claimed in claim 1 or 2, wherein the hydrocarbon feed stream comprises at least 80 %wt of iso-olefin reagent.

4. A process as claimed in any one of claims 1 to 3, wherein the iso-olefin reagent has up to 8 carbon atoms.

5. A process as claimed in any one of claims 1 to 4, wherein the alcohol reagent has up to 8 carbon atoms.

6. A process as claimed in any one of claims 1 to 5, wherein the iso-olefin reagent and the alcohol reagent are allowed to react in a molar ratio ranging from 1:0.9 to 1:1.1.

7. A process as claimed in any one of claims 1 to 6, wherein the etherification is carried out in the presence of an acidic solid catalyst.

8. A process as claimed in any one of claims 1 to 7, wherein the reaction is conducted at a temperature between 30 and 120 °C and under a pressure between 5 and 40 bar g.

9. A process as claimed in any one of claims 1 to 8, wherein the distillation is carried out in the distillation column of 30 to 50 theoretical plates.

10. A process as claimed in any one of claims 1 to 9, wherein indirect heat transfer is provided between the stream comprising alkyl tertiary-alkyl ether recovered at the bottom of the distillation column and (i) the reaction mixture and/or (ii) the hydrocarbon feed stream.

## Patentansprüche

1. Verfahren zur Herstellung eines Alkyl-tert.alkylethers durch
(a) Einspeisen eines Isoolefinreagens-hältigen Stroms und eines Alkoholreagens in einen Reaktor und Reagierenlassen dieser Reagentien unter Veretherungsbedingungen zur Ausbildung eines einen Alkyl-tert.alkylether enthaltenden Reaktionsgemisches,
(b) Überführen des in (a) gebildeten Reaktionsproduktes und eines C₄-bis C₉-Kohlenwasserstoffe enthaltenden Speisestroms (nachfolgend der "Kohlenwasserstoffspeisestrom") zu einer Destillationskolonne,
(c) Fraktionieren des Reaktionsgemisches und der Kohlenwasserstoffe zur Ausbildung eines Stromes, der im wesentlichen reinen Alkyl-tert.alkylether enthält, am Sumpf der Destillationskolonne, und eines Stromes, der Alkohol und Kohlenwasserstoffe enthält, am Kopf der Destillationskolonne, und
(d) Recyclieren wenigstens eines Teiles des Überkopfproduktes zum Reaktor,
wobei der Kohlenwasserstoffspeisestrom das Isoolefinreagens enthält, wobei wenigstens ein Teil des dem Reaktor zugeführten Isoolefinreagens-hältigen Stroms aus dem Überkopfgemisch gebildet wird, und wobei der Kohlenwasserstoffspeisestrom in die Destillationskolonne durch einen gesonderten Einlaß eingeführt wird, der unter jenem des Reaktionsgemisches angeordnet ist.

2. Verfahren nach Anspruch 1, worin wenigstens 50 Gew.-% des Isoolefinreagens-hältigen Stroms aus dem Überkopfgemisch bestehen.

3. Verfahren nach Anspruch 1 oder 2, worin der Kohlenwasserstoffspeisestrom zu wenigstens 80 Gew.-% aus Isoolefinreagens besteht.

4. Verfahren nach einem der Ansprüche 1 bis 3, worin das Isoolefinreagens bis zu 8 Kohlenstoffatome enthält.

5. Verfahren nach einem der Ansprüche 1 bis 4, worin das Alkoholreagens bis zu 8 Kohlenstoffatome enthält.

6. Verfahren nach einem der Ansprüche 1 bis 5, worin das Isoolefinreagens und das Alkoholreagens in einem Molverhältnis im Bereich von 1:0,9 bis 1:1,1 reagieren gelassen werden.

7. Verfahren nach einem der Ansprüche 1 bis 6, worin die Veretherung in Gegenwart eines sauren festen Katalysators ausgeführt wird.

8. Verfahren nach einem der Ansprüche 1 bis 7, worin die Umsetzung bei einer Temperatur von 30 bis 120°C und unter einem Druck von 5 bis 40 bar g ausgeführt wird.

9. Verfahren nach einem der Ansprüche 1 bis 8, worin die Destillation in einer Destillationskolonne mit 30 bis 50 theoretischen Böden ausgeführt wird.

10. Verfahren nach einem der Ansprüche 1 bis 9, worin zwischen dem am Boden der Destillationskolonne abgenommenen Strom, der den Alkyl-tert.alkylether enthält, und (i) dem Reaktionsgemisch und/oder (ii) dem Kohlenwasserstoffspeisestrom ein indirekter Wärmeaustausch vorgenommen wird.

## Revendications

1. Procédé de préparation d'un éther alkyl-tert-alkylique en
(a) alimentant un réacteur d'un courant contenant un réactif du type isooléfine et d'un réactif du type alcool et en permettant à ses réactifs de réagir dans des conditions d'éthérification pour former un mélange de réaction contenant un éther alkyl-tert-alkylique,
(b) faisant passer le mélange de réaction produit en (a) et un courant d'alimentation comprenant des hydrocarbures en C₄ à C₉ (que l'on appellera dans la suite du présent mémoire "courant d'alimentation en hydrocarbures") dans une colonne de distillation,
(c) fractionnant le mélange de réaction et des hydrocarbures pour obtenir un produit de queue comprenant sensiblement de l'éther alkyl-tert-alkylique pur et un mélange de tête comprenant de l'alcool et des hydrocarbures et
(d) recyclant au moins une partie du mélange de tête dans le réacteur,
où le courant d'alimentation en hydrocarbures comprend un réactif du type isooléfine, où au moins une partie du courant contenant un réactif du type isooléfine fourni au réacteur est formé par le mélange de tête, et où le courant d'alimentation en hydrocarbures est envoyé dans la colonne de distillation à travers une entrée séparée située en dessous de celle du mélange de réaction.

2. Procédé suivant la revendication 1, caractérisé en ce qu'au moins 50% du courant contenant le réactif du type isooléfine sont formés par le mélange de tête.

3. Procédé suivant la revendication 1 ou 2, caractérisé en ce que le courant d'alimentation en hydrocarbures se compose pour au moins 80% en poids du réactif du type isooléfine.

4. Procédé suivant l'une quelconque des revendications 1 à 3, caractérisé en ce que le réactif du type isooléfine comporte jusqu'à 8 atomes de carbone.

5. Procédé suivant l'une quelconque des revendications 1 à 4, caractérisé en ce que le réactif du type alcool possède jusqu'à 8 atomes de carbone.

6. Procédé suivant l'une quelconque des revendications 1 à 5, caractérisé en ce que le réactif du type isooléfine et le réactif du type alcool sont admis à réagir dans un rapport molaire qui varie de 1:0,9 à 1:1,1.

7. Procédé suivant l'une quelconque des revendications 1 à 6, caractérisé en ce que l'on entreprend l'éthérification en présence d'un catalyseur solide acide.

8. Procédé suivant l'une quelconque des revendications 1 à 7, caractérisé en ce que l'on réalise la réaction à une température comprise entre 30 et 120°C et sous une pression comprise entre 5 et 40 bars manométriques.

9. Procédé suivant l'une quelconque des revendications 1 à 8, caractérisé en ce que l'on entreprend la distillation dans une colonne de distillation comportant de 30 à 50 plateaux théoriques.

10. Procédé suivant l'une quelconque des revendications 1 à 9, caractérisé en ce que l'on opère un transfert de chaleur indirect entre le courant comprenant de l'éther alkyl-tert-alkylique récupéré au fond de la colonne de distillation et (i) le mélange réactionnel et/ou (ii) le courant d'alimentation en hydrocarbures.
